# EUROPEAN PATENT APPLICATION

(11) **EP 4 574 183 A1**
(43) Date of publication of application: **25.06.2025**
(21) Application number: 23219487.8
(22) Date of filing: 21.12.2023
(51) Int. Cl.: A61M 1/00

(54) **NEGATIVE PRESSURE WOUND THERAPY**

(71) Applicant: Mölnlycke Health Care AB, 402 52 Göteborg (SE)
(72) Inventor: UVEBORN, Johan, 436 40 Askim (SE); VANNAS, Alexander, 421 51 Göteborg (SE); LARSSON, Hanna, 421 70 Västra Frölunda (SE)
(74) Representative: Zacco Sweden AB

(57) **Abstract**

An apparatus and related aspects for providing reduced pressure to a tissue site are disclosed. The apparatus comprises a source of negative pressure configured to provide negative pressure via a first fluid flow path from an inlet of the source of negative pressure to a wound site, an electronically controllable valve configured to release negative pressure from the wound site via a second fluid flow path, a first pressure sensor configured to measure a pressure level in the first fluid flow path, and a second pressure sensor configured to measure a pressure level in the second fluid flow path. The apparatus further comprises control circuitry operatively connected to the source of negative pressure, the electronically controllable valve, the first pressure sensor, and the second pressure sensor. The control circuitry is configured to, in response to the second pressure sensor being non-responsive to a change in pressure while the source of negative pressure is active, and in response to the first pressure sensor detecting a pressure below atmospheric pressure while the source of negative pressure is active output a signal indicative of a blockage being present in the second fluid flow path. The control circuitry is further configured to, in response to the second pressure sensor being non-responsive to a change in pressure while the source of negative pressure is active, and in response to the first pressure sensor detecting a pressure level below a pressure value or detecting a pressure change rate exceeding a pressure change rate value while the source of negative pressure is active output a signal indicative of a blockage being present in the first fluid flow path.

## Description

### TECHNICAL FIELD

The herein disclosed embodiments generally relate to negative pressure wound therapy (NPWT). In particular, the herein disclosed embodiments relate to apparatuses for providing reduced pressure to a wound site, methods for controlling an apparatus for providing reduced pressure to a wound site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound site, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

The negative pressure applied to the wound site may need to be properly managed to maintain or increase the effectiveness of the negative pressure treatment. Moreover, leaks and blockages in some of the components of the NPWT apparatus may need to be detected and corrected to maintain effective treatment. For example, a leak or blockage in the tube that connects the negative pressure source to the dressing covering the wound site may disrupt the reduced pressure treatment being administered to the wound site.

### SUMMARY

The herein disclosed technology seeks to mitigate, alleviate or eliminate one or more of the above-identified deficiencies and disadvantages in the prior art to address various problems relating to blockage detection in an NPWT system.

Various aspects and embodiments of the disclosed technology are defined below and in the accompanying independent and dependent claims.

An aspect of the disclosed technology comprises an apparatus for providing negative pressure to a wound site. The apparatus comprises a source of negative pressure configured to provide negative pressure via a first fluid flow path from an inlet of the source of negative pressure to a wound site, an electronically controllable valve configured to release negative pressure from the wound site via a second fluid flow path, a first pressure sensor configured to measure a pressure level in the first fluid flow path, and a second pressure sensor configured to measure a pressure level in the second fluid flow path. The apparatus further comprises control circuitry operatively connected to the source of negative pressure, the electronically controllable valve, the first pressure sensor, and the second pressure sensor. The control circuitry is configured to, in response to the second pressure sensor being non-responsive to a change in pressure while the source of negative pressure is active, and in response to the first pressure sensor detecting a pressure below atmospheric pressure while the source of negative pressure is active output a signal indicative of a blockage being present in the second fluid flow path. The control circuitry is further configured to, in response to the second pressure sensor being non-responsive to a change in pressure while the source of negative pressure is active, and in response to the first pressure sensor detecting a pressure level below a pressure value or detecting a pressure change rate exceeding a pressure change rate value while the source of negative pressure is active output a signal indicative of a blockage being present in the first fluid flow path.

Another aspect of the disclosed technology comprises a (computer-implemented) method for operating an apparatus for providing negative pressure to a wound site. The apparatus comprises a source of negative pressure configured to provide negative pressure via a first fluid flow path from an inlet of the source of negative pressure to a wound site, and an electronically controllable valve configured to release negative pressure from the wound site via a second fluid flow path. The method comprises, in response to a pressure level in the second fluid flow path being non-responsive to a change in pressure while the source of negative pressure is active, and in response to a pressure level in the first fluid flow path being below atmospheric pressure while the source of negative pressure is active, outputting a signal indicative of a blockage being present in the second fluid flow path. The method further comprises, in response to a pressure level in the second fluid flow path being non-responsive to a change in pressure while the source of negative pressure is active, and in response to the pressure level in the first fluid flow path being below a pressure value or a pressure change rate in the first fluid flow path exceeding a pressure change rate value while the source of negative pressure is active outputting a signal indicative of a blockage being present in the first fluid flow path. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

Another aspect of the disclosed technology comprises a wound treatment system comprising apparatus according to any one of the embodiments disclosed herein, a wound cover for creating a sealed space defined in part by the wound site, and a tubing assembly defining the first fluid flow path and the second fluid flow path. With this aspect of the disclosed technology, similar advantages and preferred features are present as in the other aspects.

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that blockage conditions in NPWT systems may be detected in a reliable and robust manner.

An advantage of some embodiments is that it is possible to discern if the blockage is in an "exudate lumen" or an "air lumen" of a dual lumen (multi lumen) NPWT system.

An advantage of some embodiments is that the risk for patient discomfort caused by un-detected blockage conditions may be reduced.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2a is a schematic graph of negative pressure over time in view of a second pressure sensor for a low-leakage scenario in accordance with some embodiments.
Fig. 2b is a schematic graph of negative pressure over time in view of a first pressure sensor for a low-leakage scenario in accordance with some embodiments.
Fig. 3a is a schematic graph of negative pressure over time in view of a second pressure sensor for a high-leakage scenario in accordance with some embodiments.
Fig. 3b is a schematic graph of negative pressure over time in view of a first pressure sensor for a high-leakage scenario in accordance with some embodiments.
Fig. 4a is a schematic graph of negative pressure over time in view of a second pressure sensor for a low-leakage scenario in accordance with some embodiments.
Fig. 4b is a schematic graph of negative pressure over time in view of a first pressure sensor for a low-leakage scenario in accordance with some embodiments.
Fig. 5a is a schematic graph of negative pressure over time in view of a second pressure sensor for a high-leakage scenario in accordance with some embodiments.
Fig. 5b is a schematic graph of negative pressure over time in view of a first pressure sensor for a high-leakage scenario in accordance with some embodiments.
Fig. 6 is a schematic flowchart representation of a method for operating an apparatus for providing negative pressure to a wound site in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal.

As used herein, the term "in response to" may be construed to mean "when or "upon" or "if" depending on the context. Similarly, the phrase "if it is determined' or "when it is determined" or "in an instance of" may be construed to mean "upon determining or "in response to determining" or "upon detecting and identifying occurrence of an event" or "in response to detecting occurrence of an event" depending on the context. Accordingly, the phrase "if X equals Y" may be construed as "when X equals Y", "when it is determined that X equals Y", "in response to X being equal to Y", or "in response to detecting/determining that X equals Y" depending on the context.

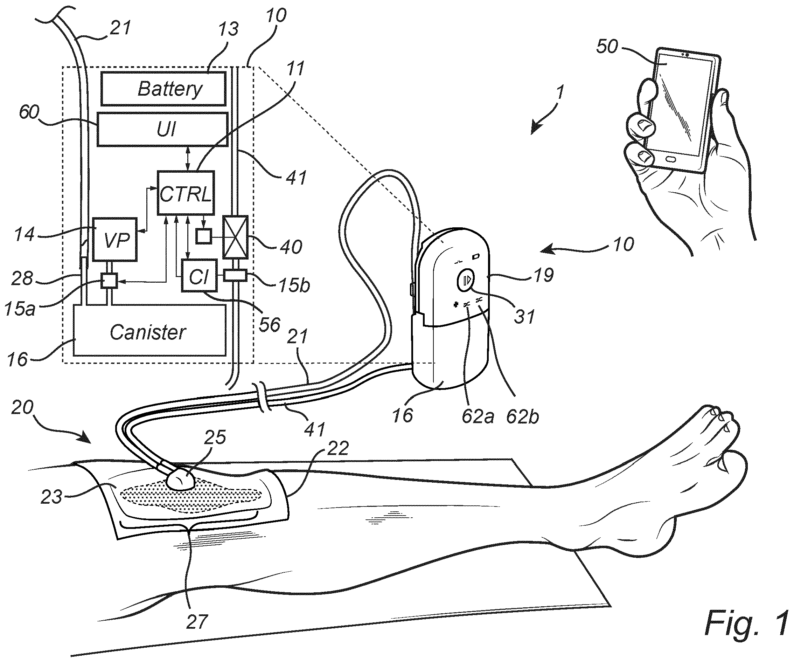

Turning now to the drawings, and to Fig. 1 in particular, there is schematically illustrated a wound treatment system 1 in accordance with some embodiments. The depicted wound treatment system 1 may be referred to as an "NPWT system" 1, "reduced pressure wound treatment system" 1, or "negative pressure wound treatment system" 1. In particular, the depicted wound treatment system 1 may be referred to as a dual lumen wound treatment system 1. The wound treatment system 1 comprises an apparatus 10 for providing negative pressure to a wound site 27 (or otherwise referred to as a tissue site 27). The wound treatment system 1 further comprises a wound cover 22 that is adapted to create a sealed space 23 defined in part by a wound surface, such as at the skin of a user/person, at or around a wound of the user/person.

Further, the apparatus 10 (may also be referred to as an NPWT device 10) is fluidly connected to the wound cover 22 using a tubing assembly with a first tubing 21 at least partly defining a first fluid flow path and a second tubing 41 at least partly defining a second fluid flow path. The tubing 21, 41 may be of any suitable flexible tubing fabricated from elastomeric or polymeric materials. The tubing 21, 41 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22. The tubing assembly may accordingly comprise two individual tubes.

The expression "wound cover" as used herein should be interpreted broadly as any wound site member, e.g. it can be a film sealed around a periphery of a wound site 27, where a wound filler may be used to fill the wound volume prior to the application of such wound cover. Wound cover may also refer to a backing layer of a wound dressing 20 comprising an additional layer(s) such as for example an absorbent layer and/or a spacer layer.

The apparatus 10 comprises a source of negative pressure 14 configured to provide negative pressure (i.e. sub-atmospheric pressure) via a first fluid flow path from an inlet of the source of negative pressure 14 to the wound dressing 20, the wound site 27, the sealed space 23, or under the wound cover 22. Thus, the term "to provide negative pressure via a first fluid flow path to a wound site" may be understood as "to provide negative pressure to a wound dressing, "to provide negative pressure to a sealed space defined by a wound cover and a wound site", or "to provide negative pressure under a wound cover". The first fluid flow path ("exudate lumen") is at least partly defined by the first tubing 21. The wound treatment system 1 further comprises a second fluid flow path that fluidly connects the wound site, or the sealed space 23 created by the wound cover 22, to the ambient atmosphere or a "fluid reservoir" (not shown) via an electronically controllable valve 40. The second fluid flow path ("air lumen") is at least partly defined by the second tubing 41. Accordingly, the fluid flow path from the inlet of the source of negative pressure 14 to the wound site 27 may be construed as a first fluid flow path or "exudate lumen", while the fluid flow path from the ambient atmosphere or fluid reservoir to the wound site 27 via the electronically controllable valve 40 may be construed as a second fluid flow path or "air lumen".

The source of negative pressure 14 is indicated as "VP" ("Vacuum pump") in the figure. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible or otherwise suitable for use in an NPWT setting and capable of maintaining or drawing adequate and therapeutic vacuum levels. Preferably, the negative pressure level to be achieved is in a range between about -20 mmHg and about -300 mmHg. In some embodiments, a negative pressure range between about -80 mmHg and about -140 mmHg is used. In some embodiments, a negative pressure range between about -115 mmHg and about -135 mmHg is used. In some embodiments, the negative pressure pump is a diaphragm pump, a peristaltic pump, piezoelectric pump or the like, in which a motor causes the moving parts to draw fluid from the wound site 27.

In the context of the present disclosure, it should be understood that the expressions "negative pressure", "sub-atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover 22 or dressing 20. In many cases, the local ambient pressure may also be the atmospheric pressure at which a patient is located. Thus, the "negative pressure" may be understood as a pressure difference between the ambient pressure and the pressure under the wound cover, where ambient pressure is generally set as 0 mmHg. Unless otherwise indicated, values of pressure stated herein are gauge pressures. Similarly, references to increases in negative pressure typically refer to a decrease in absolute/gauge pressure, while decreases in negative pressure typically refer to an increase in absolute/gauge pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user in determining the remaining capacity of the canister 16. As used herein, the term "fluidly connected" should be interpreted broadly and may comprise e.g. any form of tubing, conduits, or channels providing a fluid connection/communication between two components such as between the canister 16 and the negative pressure source 14 or the canister 16 and the wound dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14. The canister 16 may form a part of the first fluid flow path.

In some embodiments, the apparatus comprises a housing 19 enclosing the negative pressure source 14. The canister 16 may be detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments. The apparatus 10 may be of single-use type (allowing use during one single treatment time duration) or multiple-use type (allowing use during several different treatment sessions).

Furthermore, the apparatus 10 comprises a first pressure sensor 15a configured to detect, measure, or sense a pressure level in the first fluid flow path ("exudate lumen"). Moreover, the first pressure sensor 15a arranged in fluid connection with the inlet of the source of negative pressure 14 or the canister 16. In particular, the first pressure sensor 15a may be arranged proximal to the inlet of the source of negative pressure 14 (e.g., in connection with a flow path between the canister 16 and the source of negative pressure 14). In the depicted embodiment of Fig. 1, the apparatus 10 comprises a first pressure sensor 15a arranged within the housing 19 in a fluid flow path between the canister 16 and the source of negative pressure 14 (i.e., in fluid connection with the first fluid flow path). However, as readily understood by a person skilled in the art, the first pressure sensor 15a may be located at any other suitable location to sense or detect a pressure within the fluid flow path between the source of negative pressure 14 and the wound site 27. For example, the first pressure sensor 15a may be arranged within the canister.

The apparatus 10 further comprises a second pressure sensor 15b arranged in fluid connection with the second fluid flow path ("air lumen"). In particular, the apparatus 10 comprises a second pressure sensor 15b configured to detect, measure, or sense a pressure level in the second fluid flow path. In the depicted embodiment the second pressure sensor 15b is arranged within the housing 19 downstream of the electronically controllable valve 40 (i.e., towards the wound site 27 from the valve 40). However, as readily understood by the skilled person in the art, the second pressure sensors 15b may be arranged at any other suitable location to detect, measure or sense a pressure level within the second fluid flow path ("air lumen"). The first pressure sensor may be referred to as an "exudate lumen pressure sensor" and the second pressure sensor may be referred to as an "air lumen pressure sensor".

The apparatus 10 further comprises control circuitry "CTRL" 11 (may also be referred to as "a control unit", "a controller", or the like) operatively connected to the battery 13, the source of negative pressure 14, the electronically controllable valve 40, and the pressure sensors 15a, 15b. The control circuitry 11 is configured to control operation of the source of negative pressure 14 and the electronically controllable valve 40. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein. The term "electronically controllable" should in present context be understood as that one can control the unit or component that is "electronically controllable" with electric signals (control signals), and in particular with electric signals that are output from the control circuitry 11.

During use of the apparatus 10, the wound cover 22 is arranged at a wound site 27 of the user/patient, forming the sealed space 23. The tubing 21 is provided to fluidly connect the wound cover 22 to the inlet port 28 of the apparatus 10. The apparatus 10 is then activated, e.g. by a user pressing a start/pause button 31 of a user-interface 60 of the apparatus 10. The source of negative pressure 14 is thereby activated. When activated, and the valve 40 is closed, the source of negative pressure 14 will start to evacuate air through the canister 16, the inlet port 28, the tubing 21 and the sealed space 23 formed by the wound cover 22. Accordingly, a negative pressure will be created within the sealed space 23. In some embodiments, the control circuitry 11 is configured to control the source of negative pressure 14 so to establish and maintain a negative pressure level (as measured by the pressure sensors 15a, 15b) of negative pressure in a negative pressure range. It should be noted that during pressure regulation or during a pressure regulation period (maintenance of negative pressure at the wound site 27), only the output from one of the sensor's 15a, 15b may be actively used to control the pressure level. In some embodiments, only the second pressure sensor's output is used during pressure regulation. The negative pressure range may for example be -20 mmHg to -300 mmHg, -80 to -180 mmHg, -100 to -150 mmHg, - 110 to -140 mmHg, or -115 to -135 mmHg. The negative pressure range may for example be selected in view of the type of wound and/or the patient.

In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated, the duration of the treatment, as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged between at the outlet port 29 of the canister 16 to ensure that no liquid is allowed to pass to the source of negative pressure 14 from the canister 16.

The control circuitry 11 is further configured to control an operation of the electronically controllable valve 40 so to release negative pressure from the wound site via the second fluid flow path (or "air lumen") 41. In other words, the control circuitry 11 is configured to open the electronically controllable valve 40 so to introduce fluid (e.g. air) to the wound site 27 when there is a sub-atmospheric pressure under the wound cover 22. This operation may also be referred to as "flushing". Since the wound treatment system 1 depicted in Fig. 1 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1. Therefore, in order to be able to draw desired amounts of wound exudate from the wound site 27, a fluid, such as e.g. air from the ambient atmosphere, is controllably introduced at defined time intervals and/or in response to pressure measurements by opening the electronically controllable valve 40. In general, once enough fluid has been introduced (e.g. in response to a negative pressure within the first and/or second fluid flow path reaching a lower negative pressure threshold), the control circuitry 11 is configured to close the electronically controllable valve 40 and to activate the source of negative pressure 14. However, in some embodiments, the negative pressure source 14 may be active for at least some of the duration that the electronically controllable valve 40 is open.

The ability to controllably "flush" the system 1 by injecting air via an electronically controllable valve 40 may provide the advantage of longer pressure regulation cycles (may also be referred to as NPWT cycle), which reduces the on-time for the source of negative pressure 14 and therefore reduces the energy consumption of the apparatus 10.

The apparatus 10 may further comprise an air filter (not shown) arranged in the second fluid flow path in order to limit the airflow when the electronically controllable valve 40 is open. In some embodiments, the air filter comprises a hydrophobic and porous material, where the size of the pores is configured to allow for a flow in the range of 50-120 ml/s, such as 60-100 ml/s when the valve 40 is opened after negative pressure has been established under the wound cover 20. The pore size of the filter is preferably measured in a non-compressed state. In some embodiments, the air filter comprises polyethylene or sintered polyethylene. The air filter may also serve to reduce the risk of contaminants entering the wound site 27 when the valve 40 is opened (i.e., during "flushing"). The air filter may be arranged in the tube 41 that at least partly defines the second fluid flow path. The air filter may be arranged upstream of the valve 40 (i.e., between the valve 40 and the inlet of ambient air, or downstream of the valve 40 such as between the valve and the pressure sensor 15a.

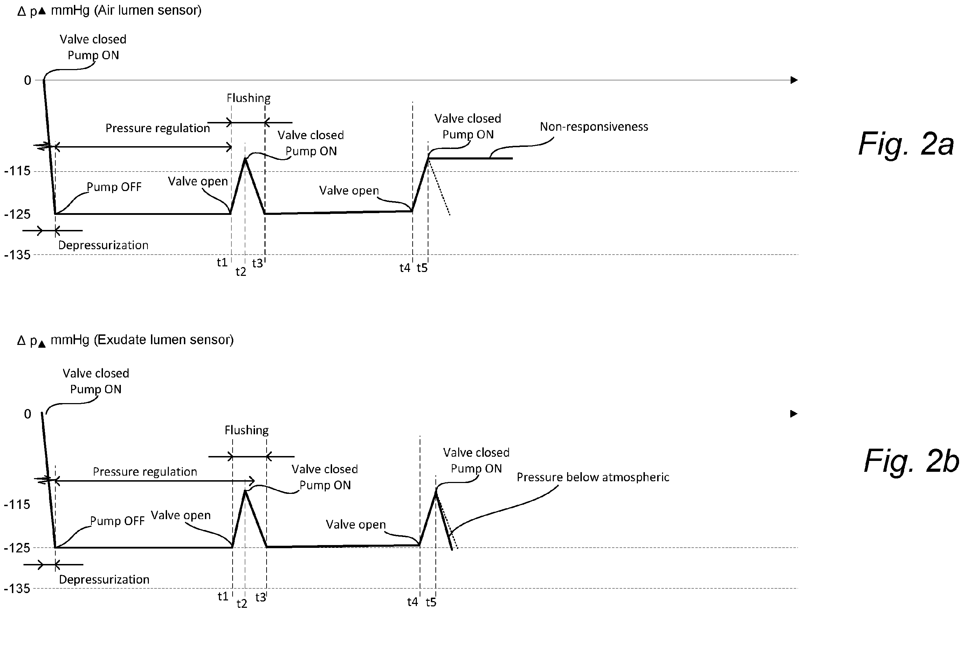

In some embodiments, during use, the control circuitry 11 is configured to regulate the negative pressure provided to the wound site over a plurality of pressure regulation cycles. In general, a pressure regulation cycle may be understood as a time period comprising at least a "pressure regulation period" and a "flushing period" (see e.g., Figs. 2a- 5b). During the "pressure regulation period" the control circuitry 11 is configured to operate the negative pressure source 14 so to maintain a level of negative pressure in a suitable negative pressure range as exemplified above, i.e., the apparatus 10 is operated in a "pressure regulation mode". In other words, during the "pressure regulation period" the electronically controllable valve 40 is closed and the source of negative pressure 14 is operated to maintain a desired level of negative pressure at the wound site 27. The desired level of negative pressure may for example comprise a negative pressure range. During the "flushing period" the control circuitry 11 is configured to open the electronically controllable valve 40 so to introduce fluid (e.g., air from the ambient atmosphere) to the wound site 27, close the valve 40, and activate the source of negative pressure 14 to draw wound exudate from the wound site and re-establish the negative pressure level, i.e., the apparatus 10 is operated in a "flushing mode". Accordingly, the flushing period causes a temporary decrease in negative pressure at the wound site 27 (i.e., a temporary increase in absolute/gauge pressure).

The durations of the pressure regulation periods and flushing periods, and accordingly the duration of a pressure regulation cycle, may be fixed (i.e., time-controlled) or dynamically controlled based pressure measurements. The duration of a pressure regulation cycle may also be dynamically controlled based on a total treatment time such that the pressure regulation cycles are shorter during an initial part of the treatment as compared to a later part. However, in some embodiments, the durations of the pressure regulation periods and flushing periods, and accordingly the duration of a pressure regulation cycle, may be controlled based on pressure measurements in combination with time-control. For example, during the flushing period the control circuitry 11 may be configured to open the valve until a pressure level P (as measured by the sensor(s) 15a, 15b) is reached or for 2 seconds, whichever occurs first. Thus, the time-control aspect may define a longest duration for each period, while detection of certain pressure levels may shorten the duration.

Moving on, the apparatus 10 is provided with a blockage-detection functionality in order to alert a user (i.e., a patient or a caregiver) of the apparatus 10 of the existence of a blockage condition in the wound treatment system 1. A blockage condition may be caused by an external impact such as a patient lying on a tube 21, 41 or a patient bending the tubing 41, 42. A blockage condition may also be caused by substances which are contained in the fluids sucked away from the wound (such as proteins) that may clog the tubing 21, 41. Even though many of the existing blockage-functionalities for dual lumen NPWT systems are capable of detecting blockage-conditions, they generally struggle with discerning if the blockage is in the air lumen or in the exudate lumen, or require additional components that add to the cost and weight of the system 1.

Accordingly, in order to detect a blockage condition and notify a user of the same, the control circuitry 11 is configured to execute a blockage-detection function where the output from the two pressure sensors 15a, 15b are used to detect whether or not there is a potential blockage in the wound treatment system 1, and if so, in which of the fluid flow paths (air lumen or exudate lumen) that the blockage is likely to be in.

It should be noted that even though the terms "exudate lumen" and "air lumen" are sometimes mentioned instead of "first fluid flow path" and "second fluid flow path", this should not be construed as that the first and second fluid flow paths are limited to solely consist of the tubes 21, 22 between the connector 25 and the apparatus 10 of the wound treatment system 1. For example, as depicted in Fig. 1 the first fluid flow path may comprise the first the connector 25, the first tubing 21, the canister 16, as well as any smaller tubing/manifolds/connectors between the canister 16 and the inlet of the source of negative pressure 14. Instead, the terms "exudate lumen" and "air lumen" are mainly used to increase readability, and could be read as "first fluid flow path" and "second fluid flow path".

In more detail, the control circuitry 11 is configured to activate the source of negative pressure 15. This may for example be done by transmitting a control signal to a motor, which in turn actuates a pump so to draw fluid from the wound site 27 via the first fluid flow path. Then, in response to the second pressure sensor 15b being non-responsive to a change in pressure while the source of negative pressure 14 is active and further in response to the first pressure sensor 15a detecting a pressure below atmospheric pressure while the source of negative pressure is active 14, the control circuitry 11 is configured to output a signal indicative of a blockage being present in the second fluid flow path. Also, in response to the second pressure sensor 15b being non-responsive to a change in pressure while the source of negative pressure 14 is active and further in response in response to the first pressure sensor 15a detecting a pressure level below a pressure value or detecting a pressure change rate exceeding a pressure change rate value while the source of negative pressure 14 is active, the control circuitry 11 is configured to output a signal indicative of a blockage being present in the first fluid flow path. In some embodiments, the above-mentioned conditions for the pressure sensors 15a, 15b further include that the electronically controllable valve 40 is closed while the source of negative pressure 14 is active.

In other words, if the air lumen pressure sensor 15b does not detect any change (or in particular any reduction) in pressure in spite of the source of negative pressure 14 being active this may be taken as an indication that a blockage condition is present in the wound treatment system 1. In some embodiments, the "non-responsiveness" of the air lumen pressure sensor 15b may comprise that the air lumen pressure sensor 15b detects a change in reduced pressure below a set value or tolerance level. In other words, the non-responsiveness need not be absolute, but may comprise a change in pressure is detected but that it is below a set value (i.e., too low of a change in pressure is being detected). Then, the exudate lumen pressure sensor 15a may be used to discern if the blockage is in the exudate lumen (first fluid flow path) or in the air lumen (second fluid flow path).

In more detail, if the exudate lumen pressure sensor 15a does indeed detect a negative pressure (within a set pressure range) while the source of negative pressure 14 is active, then it may be taken as that the blockage is present in the air lumen. This is because if the blockage is in the air lumen, the "system volume" from the perspective of the exudate lumen pressure sensor 15a is more or less the same as when there is no blockage in the system 1. Therefore, the exudate lumen pressure sensor 15a is likely to experience a normal condition, which is that it detects a negative pressure within the wound treatment system's set operating range (e.g., 0 mmHg to -150 mmHg or 0 mmHg to -135 mmHg). However, if the exudate lumen pressure sensor 15a instead detects a pressure level below a pressure value (e.g., below -150 mmHg or -135 mmHg) or a pressure change rate exceeding a pressure change rate value (e.g., 20 mmHg/s, 25 mmHg/s, 30 mmHg/s, 50 mmHg/s, 100 mmHg/s, 150 mmHg/s, or 200 mmHg/s), then it may be taken as that the blockage is present in the exudate lumen. This is because if the blockage is in the exudate lumen, the "system volume" from the perspective of the exudate lumen pressure sensor 15a is reduced as compared to when there is no blockage condition in the system (and compared to when the blockage is in the air lumen). Therefore, the exudate lumen pressure sensor 15a is likely to experience either an overshoot in the negative pressure value or a rapidly decreasing negative pressure. Furthermore, the control circuitry 11 may be configured to deactivate the source of negative pressure 14 once a blockage condition has been detected. In particular, the control circuitry 11 may be configured to deactivate the source of negative pressure 14 in response to a blockage condition being detected in the first fluid flow path, but not necessarily when a blockage condition is detected in the second fluid flow path. This is because there is a risk for patient discomfort due to excessive negative pressure value being generated due to the blockage in the exudate lumen that in turn may cause a negative pressure spike at the wound site 27. Additionally, the noise level, and in particular, the force exerted by the source of negative pressure 14 may spike in such scenarios (due to low system volume and excessive negative pressure values), which may cause damage to the internal components of the source of negative pressure 14 or other parts of the apparatus 10.

The term "in response to the second pressure sensor 15b being non-responsive to a change in pressure while the source of negative pressure is active" may be understood as "in response to the pressure level, as derived based on an output from the second pressure sensor 15b over time, being non-responsive to a change in negative pressure caused by the source of negative pressure 14", "in response to the second pressure sensor 15b not detecting/outputting a change in negative pressure, while the source of negative pressure 14 is active". As mentioned in the foregoing, the term "in response to the second pressure sensor 15b being non-responsive to a change in pressure while the source of negative pressure is active" may be include "in response to the second pressure sensor 15b detecting a change in pressure below a set value/threshold while the source of negative pressure is active". Similarly, term "in response to the first pressure sensor 15a detecting a pressure change rate exceeding a pressure change rate value" may be understood as "in response to the pressure level over time, as measured by the pressure sensor 15a over time, exceeding a pressure change rate value" or "in response to detecting a pressure change rate exceeding a pressure change rate value, where the pressure change rate is derived based on an output from the pressure sensor 15a over time". Moreover, the term "in response to the first pressure sensor 15a detecting a pressure level below a pressure value" may be understood as "in response to the pressure level, as derived based on an output from the first pressure sensor 15a over time, being below a set pressure value", or "in response to the first pressure sensor 15a detecting/outputting a pressure level below a set pressure value".

Moreover, the above exemplified pressure change rate values (20 mmHg/s, 25 mmHg/s, 30 mmHg/s, 50 mmHg/s, 100 mmHg/s, 150 mmHg/s, or 200 mmHg/s), that act as trigger values for the first pressure sensor 15a, may be selected in dependence of the type of pump/motor and the system's general settings. In more detail, if the source of negative pressure operates under lower voltages it may be assumed that the "nominal" pressure change rate, i.e., the pressure change rate while the system is operation under "normal" conditions (e.g., no blockages), is generally lower as compared to configurations where the source of negative pressure operates under higher voltages. Analogous conclusions can be drawn for pumps/motors having different operating speeds.

Thus, in some embodiments, the aforementioned "pressure change rate value" may be defined as a pressure change rate under nominal conditions (full system volume and nominal settings of the source of negative pressure) times a factor. Thus, the term "in response to the first pressure sensor 15a detecting a pressure change rate exceeding a pressure change rate value" may be understood as "in response to the first pressure sensor 15a detecting a pressure change rate exceeding a pressure change rate of N times of a nominal pressure change rate, where N is a number above 1 (i.e., N > 1)". For example, if the nominal pressure change rate for the first pressure sensor 15a is 20 mmHg/s and N=1,1, then if the pressure change rate, as detected by the first pressure sensor 15a, is above 22 mmHg/s, then the trigger condition is fulfilled. It should be noted that the 110% factor is merely one example of a trigger level, and other values such as e.g., 120%, 150%, 170%, 200% may also be used. It should also be noted that the trigger may be set as "equal to or above" rather than just "above". In other words, the term "in response to the first pressure sensor 15a detecting a pressure change rate exceeding a pressure change rate value" may be understood as "in response to the first pressure sensor 15a detecting a pressure change rate being equal to or exceeding a pressure change rate of N times of a nominal pressure change rate, where N is a number above 1 (i.e., N > 1)".

The term "while the source of negative pressure 14 is active" may be understood as, "while detecting that the source of negative pressure 14 is active", "while detecting a current draw from the source of negative pressure 14", or similar as readily understood by the skilled person in the art. Accordingly, the apparatus 10 may comprise one or more current sensors (not shown) operable to detect a current draw of the source of negative pressure 14. The control circuitry 11 may accordingly be operably connected to the current sensor, and configured to determine a state of the source of negative pressure (e.g., active or inactive). Moreover, the control circuitry 11 may be configured to detect a magnitude of current being drawn by the source of negative pressure 14 based on the output from the current sensor. However, the control circuitry 11 may also be configured to set a state of the source of negative pressure 14 (e.g., active state or inactive state) and keep track of the state of the source of negative pressure. Thereby, the term "while the source of negative pressure 14 is active" may comprise "while the set state of the source of negative pressure is an active state". Still further, the source of negative pressure 14 may be fed by a Pule Width Modulated (PWM) input signal, and the state of the source of negative pressure 14 (active state or inactive state) may be checked by monitoring the PWM signal.

The signal that is output (to indicate the presence of a blockage condition) may be a visual signal (e.g., activation of one or more LEDs on the apparatus 10), an audible signal (e.g., activation of one or more loudspeakers of the apparatus 10), and/or tactile (e.g., causing the apparatus 10 to vibrate). However, as will be elaborated in the following, the signal that is output may be a wireless signal that is transmitted to an external handheld device (e.g., smartphone or tablet), which in turn is configured to receive this signal and to output, via a Graphical User Interface (GUI) of the external handheld device, a graphical representation showing the presence of a blockage condition in the wound treatment system to a user of the external handheld device.

In some embodiments, the apparatus 10 comprises a user-interface "Ul" 60 comprising a Light Emitting Diode (LED) 62a. The user-interface 60 and the LED 62a may be operatively connected to the control circuitry 11. The LED 62a may for example be arranged on the housing 19 of the apparatus 10. The LED 62a is arranged to indicate a presence of a blockage to a user of the apparatus 10. Thus, the LED may be understood as a blockage-indicator for a user of the apparatus. Accordingly, the control circuitry 11 may be further configured to activate the LED 62a in response to the signal, indicative of a blockage in the second fluid flow path, being output, and to activate the LED 62a in response to the signal, indicative of the blockage in the first fluid flow path, being output. In other words, if a blockage is detected in either of the fluid flow paths, the LED 62a is activated so to alert a user of the apparatus 10.

Moreover, in some embodiments, the apparatus 10 comprises a user-interface 60 comprising a first LED 62a arranged to indicate a presence of a blockage being present in the first fluid flow path to a user of the apparatus 10 and a second LED 62b arranged to indicate a presence of a blockage being present in the second fluid flow path to a user of the apparatus 10. Accordingly, the control circuitry 11 may be configured to activate the second LED 62b in response to the signal, indicative of a blockage in the second fluid flow path, being output, activate the first LED 62a in response to the signal, indicative of the blockage in the first fluid flow path, being output. Using two different LEDs to discern between blockage in the "air lumen" or in the "exudate lumen" may aid the user in troubleshooting during a blockage condition.

Further, in some embodiments, the apparatus 10 further comprises a communication interface "Cl" 56 having at least one antenna and at least one transceiver operatively connected to the at least one antenna. Moreover, the transceiver may be operatively connected to the control circuitry 11, and the control circuitry 11 may be configured to transmit a first signal to an external device 50 in response to the signal, indicative of the blockage in the first fluid flow path, being output, and transmit a second signal to the external device 50 in response to the signal, indicative of a blockage in the second fluid flow path, being output. In some embodiments, the control circuitry 11 is configured to store, in a data repository, information (e.g., a log-file) about the detected blockage condition. The information may comprise time-stamped pressure values as output by the pressure sensors 15a, 15b, time-stamped activity of the source of negative pressure 14, time-stamped-activity of the valve 40, internal states of the apparatus 10 hardware, error log reports, and so forth. The stored information may subsequently be provided to the external device over a wireless or wired (serial) interface.

The communication interface 56 may comprise suitable components (e.g. transceivers, antennas, filters, power amplifiers, etc.) with suitable communication protocols (e.g., Wi-Fi, Bluetooth, Bluetooth Low Energy (BLE), Zigbee, or the like) in order to establish a connection with an external device 50 and to transmit and receive wireless signals to and from the external device 50. Accordingly, the communication interface 56 is configured to transmit and receive wireless signals to and from the external handheld device 50. In more detail, the communication interface 56 comprises one or more transceivers and one or more antennas connected to the one or more transceivers. The one or more transceivers are accordingly configured to transmit signals to an external device 50 via the one or more antennas and to receive signals from an external device 50 via the one or more antennas. For example, the communications interface 56 may include a Wi-Fi transceiver for communicating via a wireless communications network or cellular or mobile phone communications transceivers. In another example, the communication interface 56 may include a short-range wireless transmitter (e.g., a Bluetooth wireless transmitter, etc.) for communicating via a short-range wireless communication transceiver.

The external device may be an external handheld device 50, such as a mobile phone (e.g. a smart phone) operatively connected to the apparatus 10. Moreover, the handheld device 50 preferably comprises corresponding hardware and software capabilities to establish a wireless communication link with the apparatus 10 and to transmit and receive signals to and from the apparatus 10. Moreover, the external device may comprise a suitable software application configured to output a Graphical User Interface (GUI) comprising a graphical representation comprising an indication of the blockage in the wound treatment system 1 via a display apparatus of the external device 50. In more detail, depending on which signal (first or second) was received by the external device 50, the graphical representation may indicate whether the blockage is in the first fluid flow path or the second fluid flow path. Similar as before, by transmitting two different signals to the external device to discern between a blockage in the "air lumen" or in the "exudate lumen" troubleshooting may be facilitated for the user during a blockage condition.

Figs. 2a - 5b are a set of schematic graphs of negative pressure over time, Δp(t), further elucidating some embodiments disclosed herein. In particular, Figs. 2a, 3a, 4a, and 5a are schematic graphs of negative pressure over time in view of the second pressure sensor 15b, while Figs. 2b, 3b, 4b, and 5b are the corresponding schematic graphs of negative pressure over time in view of the first pressure sensor 15a in accordance with some embodiments. It should be noted that the pressure curves are schematic and not necessarily to scale, some parts have been exaggerated (e.g., the duration of the flushing period relative to the pressure regulation period, steepness of curves, etc.) to increase readability.

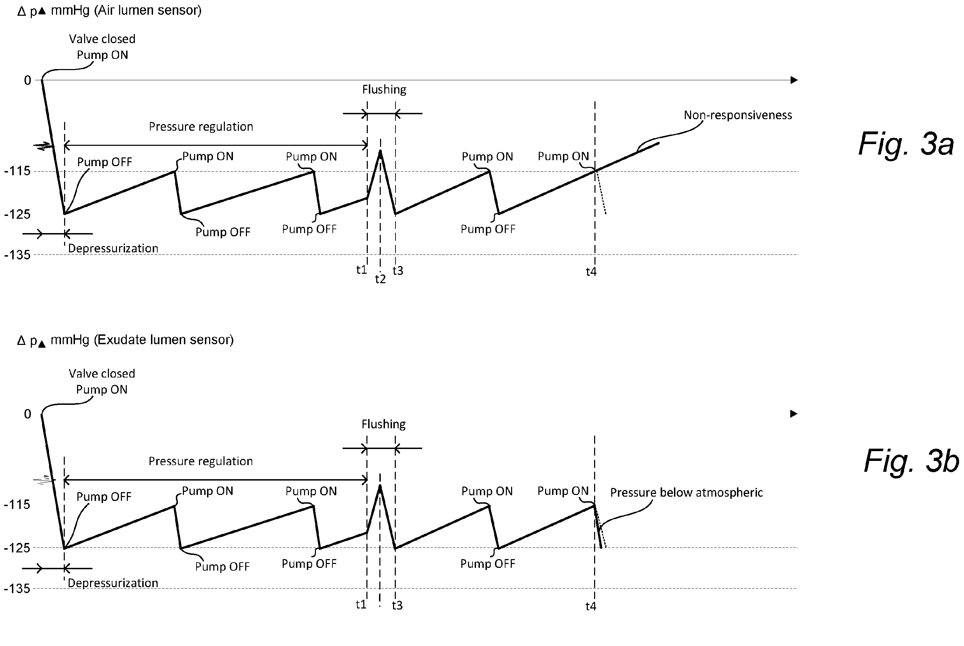

Moreover, Fig. 2a and Fig. 2b show schematic graphs of negative pressure over time in view of the second pressure sensor 15b and the first pressure sensor 15a, respectively, for a "low-leakage scenario" in accordance with some embodiments. A low-leakage scenario is in the present context to be understood that there is negligible leakage in the wound treatment system 1 as evidenced by the relatively flat pressure curve between pump activations and valve openings (e.g., between t3 and t4 in the figures). Figs. 3a and 3b depict a corresponding "elevated leakage scenario" as evidenced by the multiple starts of the pump in during the pressure regulation period. The "elevated leakage scenario" may be understood as that the wound treatment system has some unwanted leakage. The unwanted leakage may for example be due to improperly applied wound cover 22 and/or leaky connections between the tubing and other components.

As depicted in Figs. 2a, 2b, 3a, and 3b, the apparatus 10 undergoes a first depressurization (e.g., initial activation of the apparatus 10) and after which, the source of negative pressure 14 is turned off when some set negative pressure value is reached (-125 mmHg in the depicted embodiment). Then, the apparatus 10 enters a pressure regulation period or therapy regulation period where the apparatus 10 is intended to maintain a certain negative pressure at the wound site 27 for a set duration of time. This duration of time may be fixed or varied depending on the specific situation (e.g., based on the type of wound, patient, etc.). Next, the apparatus enters a flushing period, at time t1, where the electronically controllable valve 40 is opened for a duration of time or until some set pressure value is reached whereupon the valve 40 is closed and the source of negative pressure 14 is turned on, at time t2, to increase the negative pressure at the wound site 27. It should be noted that the source of negative pressure 14 may be turned on for some duration or the entire duration of the flushing period (i.e., while the valve 40 is open). Then, once the set negative pressure value is reached (-125 mmHg in the depicted embodiment) the source of negative pressure 14 is turned off. This completes a pressure regulation cycle for the apparatus 10.

However, in the subsequent pressure regulation cycle, during the flushing period, the second pressure sensor (air lumen sensor) 15b is non-responsive to a change in pressure while the source of negative pressure 14 is active (as indicated in Fig. 2a). As described in the foregoing, this is construed as an indication that a blockage condition exists. Then one can see in the pressure curve of Fig. 2b, that the first pressure sensor 15a is responsive to the change in pressure while the source of negative pressure is active, in contrast to the second pressure sensor 15b. In Figs. 3a and 3b the blockage condition trigger is detected during the pressure regulation period, and in particular, during one of the pump activations (t4 in Figs. 3a and 3b). Same as before, as indicated in Fig. 3b, the first pressure sensor 15a detects a pressure below atmospheric pressure while the second pressure sensor 15b is non-responsive. These outputs from the negative pressure sensors 15a, 15b depicted in Figs. 2a, 2b, 3a, and 3b accordingly illustrate pressure curves of a wound treatment system exhibiting a blockage condition in the second fluid flow path (air lumen) in accordance with some embodiments. Moreover, as can be seen in Figs. 3a and 3b, a blockage condition in the second fluid flow path could be detected at any activation of the source of negative pressure, and not only during the flushing period (Figs. 2a and 2b).

Moreover, the term "the first pressure sensor detecting a pressure below atmospheric pressure while the negative pressure source is active" may be understood as that "the first pressure sensor is responsive to a change in pressure while the source of negative pressure is active" or that "the first pressure sensor detects a pressure level within a pressure value range". For example, as illustrated in Figs. 2b and 3b, the pressure decrease, as detected by the first pressure sensor 15a, is within normal conditions (e.g., the pressure change rate is within a set range). Accordingly, in some embodiments, the signal indicative of a blockage being present in the second fluid flow path is output in response to the second pressure sensor 15b being non-responsive to a change in pressure while the source of negative pressure 14 is active and the first pressure sensor 15a detecting a pressure level within a pressure value range (e.g., 0 to -135 mmHg) and/or detecting a pressure change rate below a pressure change rate value (e.g., below 20 mmHg/s) while the source of negative pressure 14 is active.

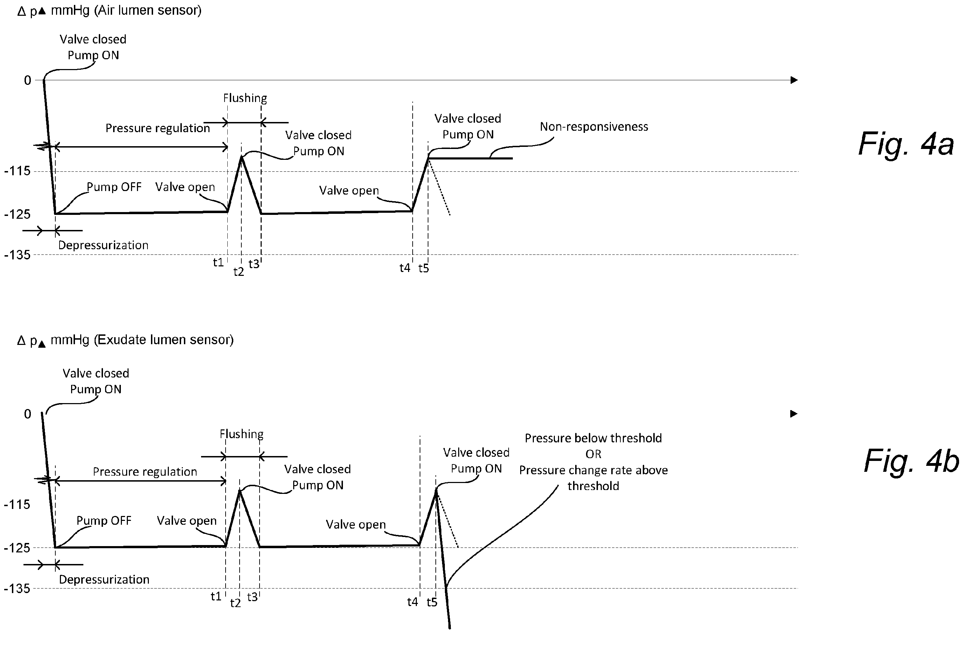

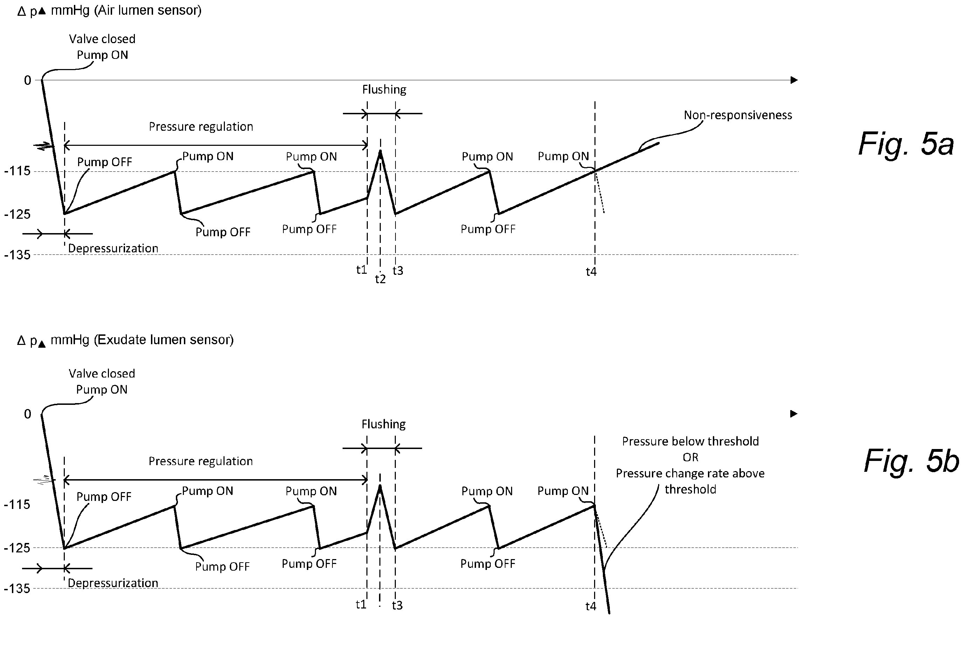

Turning to Figs. 4a, 4b, 5a, and 5c, a scenario where a wound treatment system exhibits a blockage condition in the first fluid flow path (exudate lumen) is illustrated in accordance with some embodiments. In more detail, Fig. 4a and Fig. 4b show schematic graphs of negative pressure over time in view of the second pressure sensor 15b and the first pressure sensor 15a, respectively, for a "low-leakage scenario" in accordance with some embodiments. A low-leakage scenario is in the present context to be understood that there is negligible leakage in the wound treatment system 1 as evidenced by the relatively flat pressure curve between pump activations and valve openings (e.g., between t3 and t4 in the figures). Figs. 5a and 5b depict a corresponding "elevated leakage scenario" as evidenced by the multiple starts of the pump in during the pressure regulation period. The "elevated leakage scenario" may be understood as that the wound treatment system has some unwanted leakage. The unwanted leakage may for example be due to improperly applied wound cover 22 and/or leaky connections between the tubing and other components.

As depicted in Figs. 4a, 4b, 5a, and 5b, the apparatus 10 undergoes a first depressurization (e.g., initial activation of the apparatus 10) and after which, the source of negative pressure 14 is turned off when some set negative pressure value is reached (-125 mmHg in the depicted embodiment). Then, the apparatus 10 enters a pressure regulation period or therapy regulation period where the apparatus 10 is intended to maintain a certain negative pressure at the wound site 27 for a set duration of time. This duration of time may be fixed or varied depending on the specific situation (e.g., based on the type of wound, patient, etc.). Next, the apparatus enters a flushing period, at time t1, where the electronically controllable valve 40 is opened for a duration of time or until some set pressure value is reached whereupon the valve 40 is closed and the source of negative pressure 14 is turned on, at time t2, to increase the negative pressure at the wound site 27. It should be noted that the source of negative pressure 14 may be turned on for some duration or the entire duration of the flushing period (i.e., while the valve 40 is open). Then, once the set negative pressure value is reached (-125 mmHg in the depicted embodiment) the source of negative pressure 14 is turned off. This completes a pressure regulation cycle for the apparatus 10.

However, in the subsequent pressure regulation cycle, during the flushing period, the second pressure sensor (air lumen sensor) 15b is non-responsive to a change in pressure while the source of negative pressure 14 is active (as indicated in Fig. 4a). As described in the foregoing, this is construed as an indication that a blockage condition exists. Then one can see in the pressure curve of Fig. 4b, that the first pressure sensor 15a is detecting our outputting pressure level below a pressure value (e.g., below - 150 mmHg or -135 mmHg) or detecting a pressure change rate exceeding a pressure change rate value (e.g., 20 mmHg/s, 25 mmHg/s, 30 mmHg/s, 50 mmHg/s, 100 mmHg/s, 150 mmHg/s, or 200 mmHg/s). In Figs. 5a and 5b the blockage condition trigger is detected during the pressure regulation period, and in particular, during one of the pump activations (t4 in Figs. 5a and 5b). Same as before, as indicated in Fig. 5b, the first pressure sensor 15a detects a pressure level below the pressure value or detects a pressure change rate exceeding the pressure change rate value while the second pressure sensor 15b is non-responsive. These outputs from the negative pressure sensors 15a, 15b depicted in Figs. 4a, 4b, 5a, and 5b accordingly illustrate pressure curves of a wound treatment system exhibiting a blockage condition in the first fluid flow path (exudate lumen) in accordance with some embodiments. As can be seen in Figs. 5a and 5b, a blockage condition in the first fluid flow path could be detected at any activation of the source of negative pressure, and not only during the flushing period (Figs. 4a and 4b).

The dotted parts of the curves in Figs. 2a-5b indicate the corresponding shape of the pressure curve should no blockage condition be present. It should also be noted that the length of the curves once the blockage condition trigger has been detected (i.e., after t5 in Figs. 2a, 2b, 4a, 5b and after t4 in 3a, 3b, 5a, 5b) has been exaggerated for readability. The source of negative pressure 14 may be turned off or deactivated immediately after the blockage condition trigger has been detected (e.g., within 500 ms or within 1s).

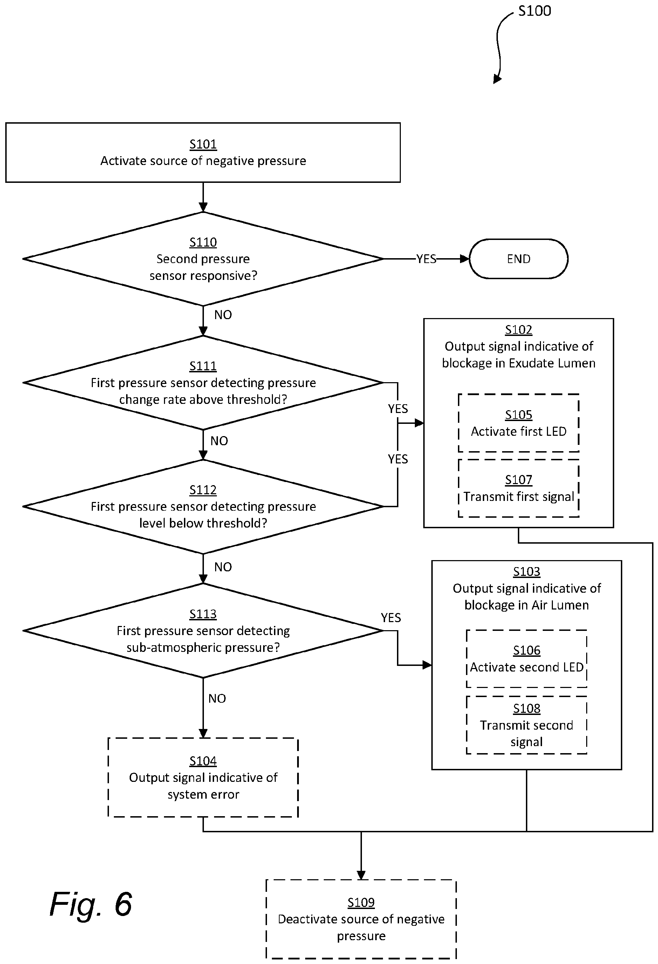

Fig. 6 is a schematic flowchart representation of a computer-implemented method S100 for operating an apparatus for providing negative pressure to a wound site in accordance with some embodiments. The apparatus may be an apparatus according to any one of the embodiments disclosed herein. However, in some embodiments, the apparatus comprises a source of negative pressure (e.g., suitable pump and actuator/motor) configured to provide negative pressure via a first fluid flow path from an inlet of the source of negative pressure to a wound site, an electronically controllable valve configured to release negative pressure from the wound site via a second fluid flow path, a first pressure sensor configured to measure a pressure level in the first fluid flow path, and a second pressure sensor configured to measure a pressure level in the second fluid flow path. The method S100 is preferably a computer-implemented method S100, performed by a processing system of the apparatus. The processing system may for example comprise one or more processors and one or more memories coupled to the one or more processors, wherein the one or more memories store one or more programs that causes the apparatus to perform the steps, services and functions of any one of the embodiments disclosed herein when executed by the one or more processors.

In some embodiments, the method S100 comprises activating S101 the source of negative pressure. In other words, the method S100 may comprise turning the source of negative pressure on so to reduce the pressure level at the wound site via the first fluid flow path. The source of negative pressure S101 may be activated S101 until a set pressure value (e.g., -125 mmHg) has been reached (as indicated by the first and/or the second pressure sensor).

Further, the method S100 comprises in response to a pressure level in the second fluid flow path being non-responsive to a change in pressure while the source of negative pressure is active, and in response to a pressure level in the first fluid flow path being below atmospheric pressure while the source of negative pressure is active, outputting S103 a signal indicative of a blockage being present in the second fluid flow path. Moreover, the method S100 comprises in response to a pressure level in the second fluid flow path being non-responsive to a change in pressure while the source of negative pressure is active, and in response to the pressure level in the first fluid flow path being below a pressure value or a pressure change rate in the first fluid flow path exceeding a pressure change rate value while the source of negative pressure is active, outputting S102 a signal indicative of a blockage being present in the first fluid flow path.

In more detail, the method S100 may be construed as a blockage detection functionality of a wound treatment system for providing negative pressure to a wound site, where a series of "if statements" are checked to detect whether or not a blockage condition is present in the wound treatment system, and then to discern whether the blockage is in the first fluid flow path or in the second fluid flow path. Thus, in some embodiments, the method S100 comprises checking S110 if the second pressure sensor is responsive to a change in pressure while the source of negative pressure is active. If it is responsive, then that is construed as that there is no blockage condition present in the system and the blockage detection function ends. However, if the second pressure sensor is non-responsive, then the method S100 comprises checking S111 if the first pressure sensor is detecting or outputting a pressure change rate above a threshold. If the first pressure sensor is detecting or outputting a pressure change rate above a threshold, then the method S100 comprises outputting S102 the signal indicative of a blockage being present in the first fluid flow path.

Similarly, if the first pressure sensor is not detecting or outputting a pressure change rate above a threshold, then the method S100 comprises checking S112 if the first pressure sensor is detecting or outputting a pressure level below a threshold value. If the first pressure sensor is detecting or outputting a pressure level below a threshold value, then the method S100 comprises outputting S102 the signal indicative of a blockage being present in the first fluid flow path. Accordingly, the checks S111, S122 of whether the first pressure sensor is detecting or outputting a pressure change rate above a threshold and if the first pressure sensor is detecting or outputting a pressure level below a threshold value are used as indicators for the blockage being in the first fluid flow path. The order of these checks S111, S112 may be alternated, and they may be used as an alternative to each other, or as complementary checks to increase robustness. Thus, in some embodiments both checks S111, S112 are used, and in some embodiments only one of the checks S111, S112 is used.

However, in the case where both of the checks S111, S112 are used, and both result in a negative (i.e., the first pressure sensor does not detect or output a pressure change rate above a threshold value and does not detect a pressure level below a threshold value), a check S113 may be performed to determine whether or not the first pressure sensor is detecting or outputting a pressure level below atmospheric pressure. Then, if the first pressure sensor is detecting or outputting a pressure level below atmospheric pressure, the method S100 comprises outputting a signal indicative of a blockage being present in the second fluid flow path. In other words, if the second pressure sensor is non-responsive, and the first pressure sensor is responsive to the change in pressure caused by the active source of negative pressure (while simultaneously not fulfilling the other conditions of elevated pressure change rate or pressure level measurement below a threshold), then this is construed as an indicator that there is a blockage in the first fluid flow path.

In some embodiments, the above-described blockage detection functionality is performed while the electronically controllable valve is closed. Thus, the method S100 may comprise closing the electronically controllable valve prior to checking S111-S113 the various pressure conditions. Moreover, in a situation where the second pressure sensor is non-responsive to the change in pressure caused by the active source of negative pressure, while the first pressure sensor fails all checks S111-S113 (e.g., the first pressure sensor is outputting a pressure value above or at atmospheric pressure, the method S100 may comprise outputting S104 a signal indicative of a system error to a user of the apparatus. The output signal S104 may be a visual, audible and/or tactile signal.

Moreover, in some embodiments, the apparatus further comprises a user-interface comprising a Light Emitting Diode (LED) arranged to indicate a presence of a blockage to a user of the apparatus. Accordingly, the outputting S103 of the signal indicative of the blockage being present in the second fluid flow path may comprise activating S106 the LED. Similarly, the outputting S102 of the signal indicative of the blockage being present in the first fluid flow path may comprise activating S105 the LED. In some embodiment the same LED is activated regardless of the blockage being in the first or the second fluid flow path. However, in some embodiments different LEDs are activated (assuming that the user-interface of the apparatus comprises two separate LEDs).

In some embodiments, the method S100 further comprises transmitting S107 a first signal to an external device in response to the signal, indicative of the blockage in the first fluid flow path, being output, and transmitting S108 a second signal to the external device in response to the signal, indicative of a blockage in the second fluid flow path, being output. Here it is assumed that the apparatus is provided with appropriate communication capabilities (software and hardware) in order to be able to transmit signals to the external device, as readily understood by the skilled person in the art.

Moreover, in some embodiments, once the signal indicative of a blockage in the first fluid flow path has been output S102, once the signal indicative of a blockage in the second fluid flow path has been output S103, or once the signal indicative of a system error S104 has been output, the method S100 further comprises deactivating S109 the source of negative pressure.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims. Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing negative pressure to a wound site, the apparatus comprising:
a source of negative pressure (14) configured to provide negative pressure via a first fluid flow path from an inlet of the source of negative pressure to a wound site;
an electronically controllable valve (40) configured to release negative pressure from the wound site via a second fluid flow path;
a first pressure sensor (15a) configured to measure a pressure level in the first fluid flow path;
a second pressure sensor (15b) configured to measure a pressure level in the second fluid flow path;
control circuitry (11) operatively connected to the source of negative pressure, the electronically controllable valve, the first pressure sensor, and the second pressure sensor, wherein the control circuitry is configured to:
in response to the second pressure sensor (15b) being non-responsive to a change in pressure while the source of negative pressure (14) is active, and
in response to the first pressure sensor (15a) detecting a pressure below atmospheric pressure while the source of negative pressure (14) is active:
output a signal indicative of a blockage being present in the second fluid flow path; and
in response to the first pressure sensor (15a) detecting a pressure level below a pressure value or detecting a pressure change rate exceeding a pressure change rate value while the source of negative pressure (14) is active:
output a signal indicative of a blockage being present in the first fluid flow path.

2. The apparatus (10) according to claim 1, wherein the control circuitry (11) is further configured to:
in response to the second pressure sensor (15b) being non-responsive to a change in pressure while the source of negative pressure (14) is active and the electronically controllable valve (40) is closed, and
in response to the first pressure sensor (15a) detecting a pressure below atmospheric pressure while the source of negative pressure (14) is active and the electronically controllable valve (40) is closed,
output the signal indicative of a blockage being present in the second fluid flow path; and
in response to the first pressure sensor (15a) detecting a pressure level below a pressure value or detecting a pressure change rate exceeding a pressure change rate value while the source of negative pressure (14) is active and the electronically controllable valve (40) is closed:
output a signal indicative of a blockage being present in the first fluid flow path.

3. The apparatus (10) according to claim 1 or 2, further comprising a user-interface (60) comprising a Light Emitting Diode, LED, (62a, 62b) arranged to indicate a presence of a blockage to a user of the apparatus (10), and wherein the control circuitry (11) is further configured to:
activate the LED (62a, 62b) in response to the signal, indicative of a blockage in the second fluid flow path, being output;
activate the LED (62a, 62b) in response to the signal, indicative of the blockage in the first fluid flow path, being output.

4. The apparatus (10) according to claim 1 or 2, further comprising a user-interface (60) comprising a first LED (62a) arranged to indicate a presence of a blockage being present in the first fluid flow path to a user of the apparatus and a second LED (62b) arranged to indicate a presence of a blockage being present in the second fluid flow path to a user of the apparatus (10), and wherein the control circuitry (11) is configured to:
activate the second LED (62b) in response to the signal, indicative of a blockage in the second fluid flow path, being output;
activate the first LED (62a) in response to the signal, indicative of the blockage in the first fluid flow path, being output.

5. The apparatus (10) according to any one of claims 1-4, further comprising a communication interface (56) having at least one antenna and at least one transceiver operatively connected to the at least one antenna; and
wherein the control circuitry (11) is operatively connected to the at least one transceiver and configured to:
transmit a first signal to an external device (50) in response to the signal, indicative of the blockage in the first fluid flow path, being output; and
transmit a second signal to the external device in response to the signal, indicative of a blockage in the second fluid flow path, being output.

6. The apparatus (10) according to any one of claims 1-5, further comprising a current sensor configured to detect a current draw of the source of negative pressure, wherein the control circuitry (11) is operatively connected to the current sensor and configured to detect whether the source of negative pressure (14) is active or inactive based on an output from the current sensor.

7. The apparatus (10) according to any one of claims 1-6, wherein the control circuitry (11) is further configured to:
in response to the second pressure sensor (15b) being non-responsive to a change in pressure while the source of negative pressure (14) is active, and
in response to the first pressure sensor (15a) detecting a pressure level below a pressure value or detecting a pressure change rate exceeding a pressure change rate value while the source of negative pressure (14) is active:
deactivate the source of negative pressure (14).

8. A method (S100) for operating an apparatus for providing negative pressure to a wound site, wherein the apparatus comprises a source of negative pressure configured to provide negative pressure via a first fluid flow path from an inlet of the source of negative pressure to a wound site, and an electronically controllable valve configured to release negative pressure from the wound site via a second fluid flow path, the method (S100) comprising:
in response to a pressure level in the second fluid flow path being non-responsive to a change in pressure while the source of negative pressure is active, and
in response to a pressure level in the first fluid flow path being below atmospheric pressure while the source of negative pressure is active:
outputting (S103) a signal indicative of a blockage being present in the second fluid flow path; and
in response to the pressure level in the first fluid flow path being below a pressure value or a pressure change rate in the first fluid flow path exceeding a pressure change rate value while the source of negative pressure is active:
outputting (S102) a signal indicative of a blockage being present in the first fluid flow path.

9. The method (S100) according to claim 8, further comprising:
in response to a pressure level in the second fluid flow path being non-responsive to a change in pressure while the source of negative pressure is active and the electronically controllable valve is closed, and
in response to a pressure level in the first fluid flow path being below atmospheric pressure while the source of negative pressure is active and the electronically controllable valve is closed:
outputting (S103) the signal indicative of a blockage being present in the second fluid flow path; and
in response to the pressure level in the first fluid flow path being below a pressure value or a pressure change rate in the first fluid flow path exceeding a pressure change rate value while the source of negative pressure is active and the electronically controllable valve is closed:
outputting (S102) the signal indicative of a blockage being present in the first fluid flow path.

10. The method (S100) according to claim 8 or 9, wherein the apparatus further comprises a user-interface comprising a Light Emitting Diode, LED, arranged to indicate a presence of a blockage to a user of the apparatus;
wherein outputting (S103) the signal indicative of the blockage being present in the second fluid flow path comprises activating the LED; and
wherein outputting (S102) the signal indicative of the blockage being present in the first fluid flow path comprises activating the LED.

11. The method (S100) according to any one of claims 8 or 9, wherein the apparatus further comprises a user-interface comprising a first LED arranged to indicate a presence of a blockage being present in the first fluid flow path to a user of the apparatus and a second LED arranged to indicate a presence of a blockage being present in the second fluid flow path to a user of the apparatus:
wherein outputting (S103) the signal indicative of the blockage being present in the second fluid flow path comprises activating (S106) the second LED;
wherein outputting (S102) the signal indicative of the blockage being present in the first fluid flow path comprises activating (S105) the first LED.

12. The method (S100) according to any one of claims 8-11, further comprising:
transmitting (S107) a first signal to an external device in response to the signal, indicative of the blockage in the first fluid flow path, being output; and
transmitting (S108) a second signal to the external device in response to the signal, indicative of a blockage in the second fluid flow path, being output.

13. The method (S100) according any one of claims 8-12, further comprising:
in response to the second pressure sensor being non-responsive to a change in pressure while the source of negative pressure is active, and
in response to the first pressure sensor detecting a pressure level below a pressure value or detecting a pressure change rate exceeding a pressure change rate value while the source of negative pressure is active:
deactivating (S109) the source of negative pressure.

14. A computer program product comprising instructions which, when the program is executed by a computing device of an apparatus for providing negative pressure to a wound site, causes the apparatus to carry out the method (S100) according to any one of claims 8-13.

15. A system (1) comprising:
an apparatus (10) according to any one of claims 1-7;
a wound cover (22) for creating a sealed space defined in part by the wound site; and
a tubing assembly defining the first fluid flow path and the second fluid flow path.
